(19) Europäisches Patentamt
European Patent Office
Office européen des brevets

(11) Publication number: **0 296 717**
**A2**

(12) # EUROPEAN PATENT APPLICATION

(21) Application number: 88304727.6

(51) Int. Cl.[4]: **C07H 17/08 , A61K 31/70**

(22) Date of filing: 25.05.88

Claims for the following Contracting States: ES + GR.

(30) Priority: 26.05.87 US 53641

(43) Date of publication of application: 28.12.88 Bulletin 88/52

(84) Designated Contracting States: AT BE CH DE ES FR GB GR IT LI NL SE

(71) Applicant: **ELI LILLY AND COMPANY**
**Lilly Corporate Center**
**Indianapolis Indiana 46285(US)**

(72) Inventor: **Kirst, Herbert Andrew**
**1819 Madison Village Drive Apartment B-6**
**Indianapolis Indiana 46227(US)**
Inventor: **Wind, Julie Ann**
**898 Dover Drive, Apt. 909**
**Greenwood Indiana 46142(US)**

(74) Representative: **Hudson, Christopher Mark et al**
**Erl Wood Manor**
**Windlesham Surrey GU20 6PH(GB)**

(54) **Ring-contracted macrolides.**

(57) Novel 12-membered lactone and 11-membered lactone derivatives of erythromycin, having antimicrobial activity against certain Gram-positive pathogens such as Streptococcus pyogenes and Gram-negative cocci such as Haemophilus influenzae, and useful as intermediates to other macrolide derivatives, are disclosed. They have the formula (A)

EP 0 296 717 A2

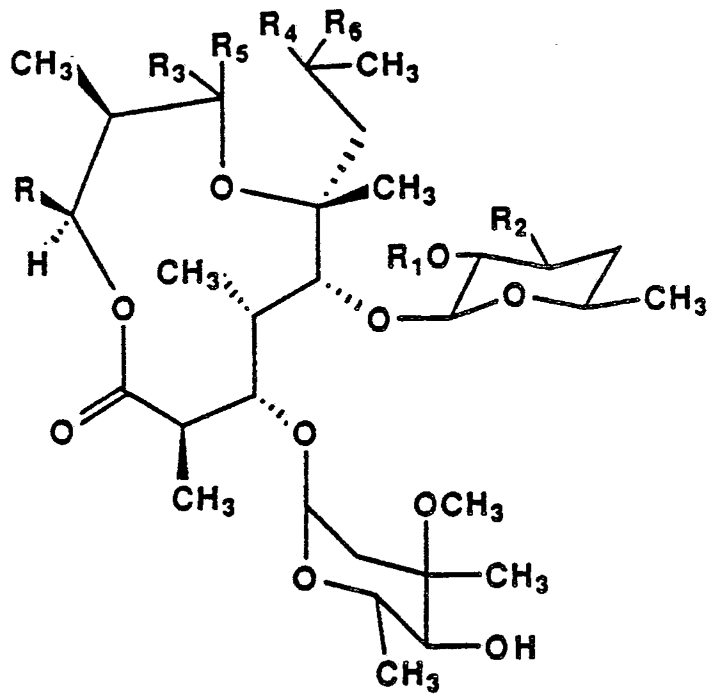

(A)

wherein

R is a) 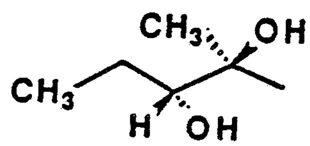 or b) acetyl;

$R^1$ is hydrogen or $C_1$-$C_5$-alkanoyl;
$R^2$ is $-N(CH_3)_2$ or $-N(CH_3)_2 \rightarrow O$; and
either

a) $R^5$ and $R^6$ taken together form a bond, and $R^3$ and $R^4$ are 1) either both hydroxyl or 2) taken together form a bond; or

b) each of $R^3$ and $R^5$ taken together and $R^4$ and $R^6$ taken together form a keto group;

provided that, when R is an (a) group and $R^2$ is $-N(CH_3)_2$, both $R^3$ and $R^4$ together and $R^5$ and $R^6$ together cannot form a bond; or a salt thereof.

## RING-CONTRACTED MACROLIDES

This invention relates to novel macrolide antibiotics, which are 12-membered lactone and 11-membered dilactone derivatives of erythromycin, and to the salts and ester derivatives of these compounds.

New, improved antibiotics are continually in demand. In addition to antibiotics which are useful for treating human diseases, improved antibiotics are also needed in the veterinary field. Increased potency, expanded spectrum of bacterial inhibition, increased in vivo efficacy, and improved pharmaceutical properties (such as greater oral absorption, higher blood or tissue concentrations, longer body half life, and more advantageous rate or route of excretion and rate or pattern of metabolism) are some of the goals for improved antibiotics.

The macrolide antibiotic erythromycin has been the subject of much study, and a number of interesting derivatives such as erythromycylamine, 6-0-methylerythromycin and 8-fluoroerythromycin have been prepared. Making changes in the size of the macrolide ring itself, however, has not been extensively reported. Thus, it was quite surprising to discover methods for making the ring-contracted erythromycin derivatives of this invention.

The new ring-contracted derivatives of this invention have the structure shown in the formula 1.

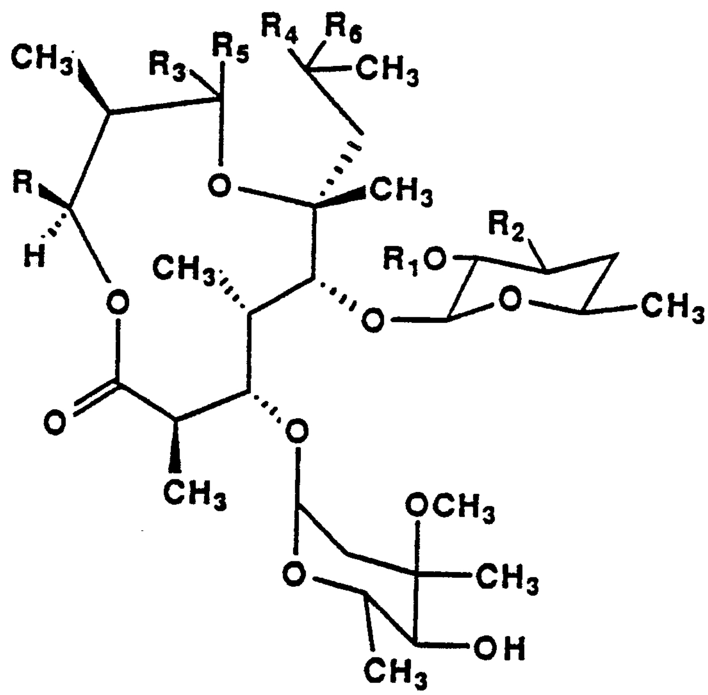

wherein

R is (a) or (b) acetyl;

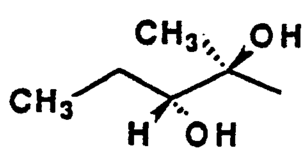

$R^1$ is hydrogen or $C_1$-$C_5$-alkanoyl;
$R^2$ is $-N(CH_3)_2$ or $-N(CH_3)_2 \rightarrow O$; and
either

a) $R^5$ and $R^6$ taken together form a bond, and $R^3$ and $R^4$ are 1) either both hydroxyl or 2) taken together form a bond; or

b) each of $R^3$ and $R^5$ taken together and $R^4$ and $R^6$ taken together form a keto group;

provided that, when R is an (a) group and $R^2$ is $-N(CH_3)_2$, both $R^3$ and $R^4$ together and $R^5$ and $R^6$ together cannot form a bond; and their salts.

Thus, one group of formula 1 compounds has formula 1a

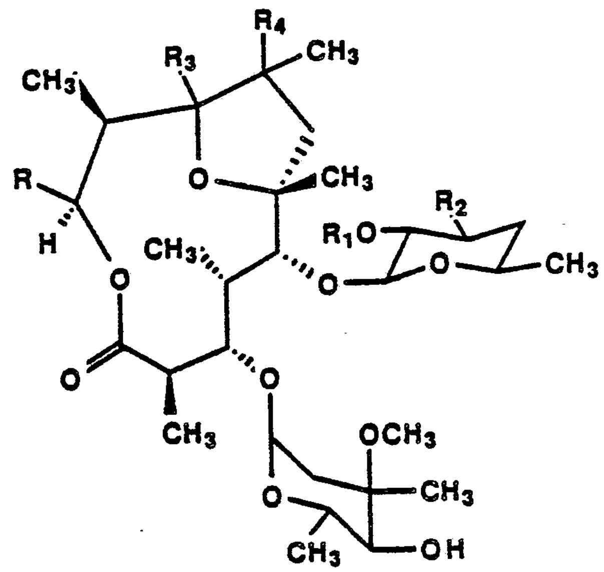

1a

wherein R, $R^1$ and $R^2$ are as defined, and $R^3$ and $R^4$ are either both hydroxyl or taken together form a bond; provided that, when R is an (a) group and $R^2$ is $-N(CH_3)_2$, $R^3$ and $R^4$ must both be hydroxyl; or a salt thereof.

The other group of formula 1 compounds have formula 1b:

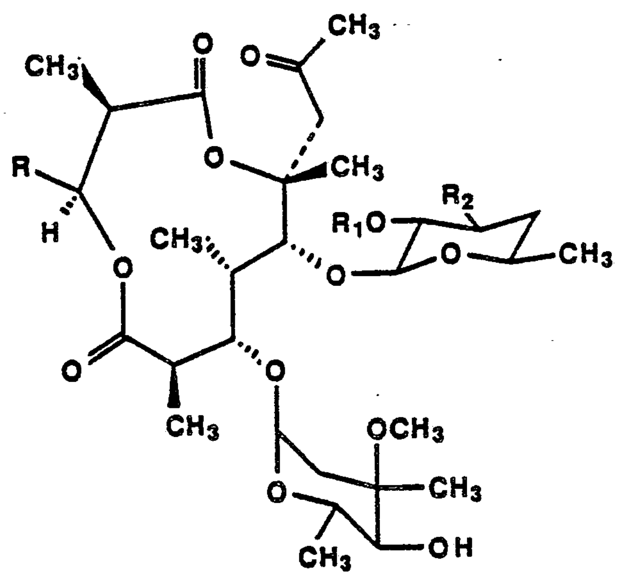

1b

wherein

R, $R^1$ and $R^2$ are as defined supra; and their salts.

Figure 1 shows the reaction sequences used to prepare Formula 1a compounds, and Figure 2 shows the reaction sequences used to prepare Formula 1b compounds.

Acid-catalyzed conversion of erythromycin to its 8,9-anhydro-6,9-hemiketal derivative (compound 2) is well known.[4] The lactone carbonyl group in this enol ether derivative (2) can migrate from the C-13 hydroxyl to the C-11 hydroxyl group under a wide variety of reaction conditions to yield a 12-membered ring enol ether derivative (see Figure 2 - compound 3). This trans-lactonization process occurs under a variety of both acidic and basic conditions as well as thermally (in refluxing toluene). Furthermore, the acyl migration is reversible in many of these cases, so that an equilibrium between compounds 2 and 3 is established.

A preferred method for preparing compound 3 from compound 2 uses potassium carbonate in refluxing methanol. This method gives a mixture of compound 3 and compound 2 in a ratio of approximately 6:1 (HPLC analysis); however, isolation of compound 3 on a multi-gram scale is relatively easy, using well known procedures such as extraction and chromatography.

Unfortunately, trans-lactonization using potassium carbonate in refluxing methanol has been confined to the enol ether 2. Erythromycin itself as well as erythromycylamine, erythromycin-9-hydrazone, erythromycin anhydro-6,9;9,12-spiroketal and 9-dihydroerythromycin all failed to give any detectable conversion to ring-contracted products.

The transformation of compound 2 to compound 3 has been accomplished by conditions as diverse as 1) potassium carbonate in relfuxing toluene or tetrahydrofuran (THF), 2) triethylamine in refluxing methanol, 3) 9-borabicyclo[3.3.1]nonane (9-BBN) in THF, 4) mercuric acetate in methanol and 5) iron pentacarbonyl in refluxing toluene, with trans-lactonization being the only apparent reaction.

The formula 1 compounds wherein R is acetyl are prepared by selectively cleaving the diol tail from those formula 1 compounds wherein R is (a). Selective cleavage can be accomplished using lead tetra-acetate in inert solvents such as toluene.

The formula 1 compounds wherein $R^2$ is $-N(CH_3)_2 \rightarrow O$ are prepared by oxidizing the formula 1 compounds wherein $R^2$ is $-N(CH_3)_2$. Hydrogen peroxide or peracids such as m-chloroperbenzoic acid (MCPBA) are preferred oxidizing agents. The reverse transformation, i.e. $-NMe_2 \rightarrow O$ to $-NMe_2$, can be achieved by reducing agents such as phosphorous(III) reagents (e.g. triphenylphosphine and tributylphosphine) or trialkylboranes (e.g. $(sec\text{-}Bu)_3B$).

The compounds of formula 1a wherein $R^3$ and $R^4$ are both hydroxyl are prepared by oxidizing the double bond in those formula 1a compounds wherein $R^3$ and $R^4$ together form a bond. Suitable oxidizing agents for this reaction are bromine, N-bromosuccinimide or N-chlorosuccinimide in solvents such as aqueous acetontrile.

The compound of formula 1b wherein R is (a), $R^1$ is hydrogen and $R^2$ is $-N(CH_3)_2 \rightarrow O$ is prepared by treating the ring-contracted enol ether compound (3) with m-chloroperbenzoic acid in dichloromethane at 0 °C. This reaction gives a mixture of products from which the 11-membered-ring diolide N-oxide can be isolated as the principal component, albeit in low yield.

The compound of formula 1b wherein R is acetyl, $R^1$ is hydrogen and $R^2$ is $-N(CH_3)_2$ is prepared by treating compound 3 with sodium periodate in aqueous acetonitrile.

The derivatives of this invention wherein $R^2$ is $-N(CH_3)_2$ can form salts, particularly acid addition salts. These acid addition salts are also useful as antibiotics and are a part of this invention. In another aspect, such salts are useful as intermediates, for example, for separating and purifying the derivatives. In addition, the salts have an improved solubility in water.

Representative suitable salts include those salts formed by standard reactions with both organic and inorganic acids such as, for example, sulfuric, hydrochloric, phosphoric, acetic, succinic, citric, lactic, maleic, fumaric, palmitic, cholic, pamoic, mucic, D-glutamic, d-camphoric, glutaric, glycolic, phthalic, tartaric, formic, lauric, stearic, salicylic, methanesulfonic, benzenesulfonic, sorbic, picric, benzoic, cinnamic, and like acids.

Pharmaceutically acceptable acid addition salts are an especially prefered group of salts of this invention. Pharmaceutically acceptable acid addition salts are those salts useful in the chemotherapy of a warm-blooded animal.

The compounds of formula 1 wherein $R^1$ is $C_1$-$C_5$-alkanoyl are prepared by esterifying the appropriate 1 compounds wherein $R^1$ is hydrogen by treatment with acylating agents, using standard methods well exemplified in the art (see, for example, Baltz et al. in U.S. Patent 4,321,361).

The new derivatives of this invention have antibacterial activity, but should be most valuable as intermediates to novel antibacterial agents.

The formula 1 compounds inhibit the growth of certain pathogenic bacteria, especially Gram-positive

bacteria and Gram-negative cocci such as Haemophilus influenzae. Table I summarizes the minimal inhibitory concentrations (MIC's) at which these compounds inhibit certain organisms, as determined by standard agar-dilution assays.

Table I: Antibiotic Activity of Ring Contracted Derivatives[a]

| Organism | Compound Number[b] 6 | 8 |
|---|---|---|
| Staphylococcus aureus X1.1 | 128 | --- |
| Staphylococcus aureus V41[c] | --- | --- |
| Staphylococcus epidermidis 270 | --- | --- |
| Staphylococcus epidermidis 222 | 64 | --- |
| Streptococcus pyogenes C203 | 16 | 128 |
| Streptococcus pneumoniae Park I | 8 | 32 |
| Streptococcus sp. X66 | 16 | 64 |
| Streptococcus sp. group D 2041 | 64 | --- |
| Haemophilus influenzae C.L.[d] | 8 | 64 |
| Haemophilus influenzae 76[e] | 8 | 64 |

[a]MIC's in mcg/mL

[b]Compound numbers from Figures 1 and 2

[c]Penicillin-resistant strain

[d]Ampicillin-sensitive strain

[e]Ampicillin-resistant strain

This invention also includes 1) a formula 1 compound, or a pharmaceutically acceptable salt thereof, for use in inhibiting bacteria and 2) pharmaceutical formulations which comprise as an active ingredient, a formula 1 compound, or a pharmaceutically acceptable salt thereof, associated with one or more pharmaceutically acceptable carriers.

The following examples are provided in order to illustrate this invention.

Product purification by chromatography was performed on silica gel, using either flash chromatography techniques (E. Merck grade 60 silica gel, 230-400 mesh) or a Waters Model 500 Prep LC system.

Compounds were purified to homogeneity according to thin layer chromatographic (TLC) and proton NMR analyses.

## Preparation 1

8,9-Anhydro-erythromycin-6,9-hemiketal (Compound 2)

A solution of erythromycin (20.0 g, 27.3 mmol) in glacial acetic acid (100 ml) was stirred at room temperature for 1 hour. Sodium hydroxide 5N was slowly added until precipitation was complete after the mixture had cooled back to ambient temperature. The mixture was extracted twice with dichloromethane. The combined organic layers were extracted with saturated sodium bicarbonate solution, dried (sodium sulfate), filtered and evaporated. The crude produce (18.9 g) was purified by preparative HPLC (linear gradient of dichloromethane to 7% methanol + 0.5% ammonium hydroxide in dichloromethane) to yield Compound 2 (13.2 g, 68%) as a white solid.

## Preparation 2

Compound 3 from Trans-lactonization of Compound 2

Compound 2 (10.0 g, 14 mmol) in methanol (200 mL) was treated with potassium carbonate (1.9 g, 14 mmol), and the mixture was refluxed for 90 min. Solvent was evaporated under reduced pressure, and the residue was partitioned between dichloromethane and saturated sodium bicarbonate solution. The organic layer was evaporated to give 9.6 g of a white foam. This foam was purified by preparative HPLC (linear gradient of dichloromethane to 7.5% methanol + 0.5% ammonium hydroxide in dichloromethane) to yield Compound 3 (5.4 g, 54%) as a white solid. FDMS m/e 715 (M + H).

## Preparation 3

Compound 4 from Lead Tetraacetate Cleavage of Compound 3

Compound 3 (2.0 g, 2.8 mmol) was dissolved in toluene (80 ml) and treated with lead tetra-acetate (1.9 g, 4.2 mmol). After being stirred at room temperature for 50 min., the heterogeneous mixture was extracted twice with saturated sodium bicarbonate solution, dried (sodium sulfate), filtered and evaporated. The crude produce (1.8 g) was separated by flash chromatography, eluting with a gradient of dichloromethane to dichloromethane-methanol-ammonium hydroxide (96:4:0.5), to give compound 4 (780 mg, 43%) as a white foam. FDMS m/e 655 (M + H); IR 1720 $cm^{-1}$ (ketone carbonyl).

## Example 1

Compound 5 from N-Oxidation of Compound 3

Compound 3 (100 mg 0.14 mmol) was dissolved in acetonitrile (1 ml) and water (0.5 ml) and then treated with 30% Hydrogen peroxide (0.014 ml) dropwise. The reaction was stirred at room temperature for 2 days, during which a white solid precipitated. The heterogenous mixture was partitioned between dichloromethane and saturated sodium bicarbonate solution. The organic layer was dried (sodium sulfate) and evaporated to give 60 mg (59%) of Compound 5. $^1H$ NMR was like that of Compound 3 except: δ 4.45-(1′), 3.76(2′), 3.39(3′), 1.96/1.38(4′), 3.59(5′), 1.27(5′-$CH^3$), 3.20($NMe^2$); FDMS m/e 731 (M + H).

## Example 2

Compound 6 from Oxidation of Compound 3

Compound 3 (100 mg 0.14 mmol) was dissolved in acetonitrile (1 mL) and water (0.g ml) and cooled to 0 °C for 15 min. A solution of bromine (23 mg, 0.14 mmol) in water (1 ml) was added dropwise. After being stirred for 20 min. at 0 °C, the reaction mixture was partitioned between dichloromethane and saturated sodium bicarbonate solution. The organic layer was dried (sodium sulfate), filtered and evaporated to give 85 mg of Compound 6 (81%) as a white solid. FDMS m/e 749 (M + H).

## Example 3

Diolide 7 from MCPBA Cleavage of Compound 3

Compound 3 (1.0 g 1.4 mmol) was dissolved in dichloromethane (10 ml) and cooled at 0 °C for 30 min. A solution of m-chloroperbenzoic acid (80%, 870 mg, 0.42 mmol) was added dropwise to the cooled solution. Since conversion was incomplete after 2 hr. at 0 °C (TLC), additional m-chloroperbenzoic acid (435 mg, 0.21 mmol) in dichloromethane (5 ml) was added. After an additional 2 hr., no change was apparent by TLC. The mixture was extracted with 10% sodium bisulfite solution and then with saturated sodium bicarbonate solution. The organic layer was dried (sodium sulfate) and evaporated to give 390 mg of crude produce, from which 98 mg (9%) of Compound 7 was obtained by crystallization from dichloromethane. FDMS m/e 764 $(M + H)^+$; IR 1723 $cm^{-1}$ (lactone carbonyl).

## Example 4

Diolide 8 from Sodium Periodate Cleavage of Compound 3

Compound 3 (100 mg 0.14 mmol) was dissolved in methanol (1 ml) and water (0.5 ml). Sodium periodate (240 mg, 1.12 mmol) was dissolved in water (3 ml), with the aid of sonication and methanol (2 ml) and was then added dropwise, yielding a white precipitate. After stirring the heterogeneous mixture for 11 days at room temperature, it was partitioned between ethyl acetate and saturated sodium bicarbonate solution. The crude produce (60 mg) was purified by flash chromatography, eluting with a gradient of dichloromethane to dichloromethane - methanol (23:2), to yield Compound 8 (45 mg, 47%) as a colorless glass. FDMS m/e 687 (M+); IR 1727 $cm^{-1}$ (lactone carbonyl).

Table II. Proton NMR Chemical Shifts of Macrolide Derivatives[a]

| Position | 2 | 3 | 4 | 6 | 7 | 8 |
|---|---|---|---|---|---|---|
| 2 | 2.74 | 2.83 | 2.80 | 2.81 | 2.81 | 2.85 |
| 3 | 4.09 | 4.29 | 4.23 | 4.00 | 4.08 | 4.03 |
| 4 | 1.88 | 1.7 | 1.72 | 2.02 | 2.14 | 2.13 |
| 5 | 3.89 | 3.70 | 3.67 | 3.48 | 3.74 | 3.80 |
| 7 | 2.65/1.97 | 2.78/2.03 | 2.75/2.01 | 2.81/2.00 | 3.15/2.84 | 3.18/3.02 |
| 10 | 2.79 | 2.93 | 3.19 | 3.04 | 2.84 | 3.13 |
| 11 | 3.47 | 5.06 | 5.25 | 5.02 | 5.25 | 5.44 |
| 13 | 4.86 | 2.83 | -- | 3.03 | 2.98 | -- |
| 13-$CH_2$ | 1.88/1.47 | ~1.6/~1.3 | -- | 1.72/1.24 | 1.89/1.31 | -- |
| 13-$CH_3$ | 0.88 | 0.98 | -- | 1.02 | 0.97 | -- |
| 2-$CH_3$ | 1.15 | 1.27 | 1.30 | 1.23 | 1.28 | 1.35 |
| 4-$CH_3$ | 1.10 | 1.10 | 1.07 | 1.11 | 1.12 | 1.07 |
| 6-$CH_3$ | 1.35 | 1.42 | 1.39 | 1.52 | 1.76 | 1.77 |
| 8-$CH_3$ | 1.57 | 1.55 | 1.55 | 1.37 | 2.19 | 2.15 |
| 10-$CH_3$ | 1.06 | ~1.2 | 0.98 | 1.17 | 1.37 | 1.15 |
| 12-$CH_3$ | 1.06 | ~1.2 | 2.09 | 1.32 | 1.14 | 2.15 |
| 1' | 4.44 | 4.33 | 4.31 | 4.23 | 4.46 | 4.40 |
| 2' | 3.21 | 3.20 | 3.17 | 3.25 | 3.66 | 3.18 |
| 3' | 2.44 | 2.48 | 2.46 | 2.56 | 3.34 | 2.45 |
| 4' | 1.68/1.26 | ~1.6/~1.2 | 1.62/1.25 | 1.75/1.24 | 1.89/1.31 | 1.68/~1.20 |
| 5' | 3.52 | 3.48 | 3.44 | 3.46 | 3.56 | 3.48 |
| 5'$CH_3$ | 1.24 | 1.19 | 1.22 | 1.20 | 1.23 | 1.22 |
| $N(CH_3)_2$ | 2.29 | 2.29 | 2.26 | 2.32 | 3.16/3.14 | 2.29 |
| 1" | 5.09 | 4.89 | 4.90 | 4.77 | 4.90 | 4.93 |
| 2" | 2.41/1.60 | 2.38/~1.5 | 2.35/1.55 | 2.33/1.54 | 2.35/1.53 | 2.36/1.57 |
| 4" | 3.06 | 3.03 | 3.00 | 3.00 | 3.01 | 3.02 |
| 5" | 4.09 | 4.05 | 4.02 | 4.05 | 3.94 | 3.99 |

Table II. Proton NMR Chemical Shifts of Macrolide Derivatives[a] (Continued)

| Position | 2 | 3 | 4 | 6 | 7 | 8 |
|---|---|---|---|---|---|---|
| 5"-$CH_3$ | 1.32 | 1.33 | 1.30 | 1.23 | 1.27 | 1.29 |
| 3"-$CH_3$ | 1.26 | 1.21 | 1.22 | 1.20 | 1.23 | 1.24 |
| 3-$OCH_3$ | 3.36 | 3.29 | 3.26 | 3.23 | 3.29 | 3.28 |
| OH3.09 | NA[b] | NA | NA | NA | NA | NA |
| 4"-OH | 2.19 | NA | NA | NA | NA | NA |

a Obtained in deuteriochloroform solution using a Bruker WM-270 NMR spectrometer; chemical shifts are reported in parts per million from internal tetramethylsilane.

b Number of the carbon atoms in all compounds corresponds to their respective initial positions in 2.

c NA means not assigned

Table III. C-13 NMR Chemical Shifts of Macrolide Derivatives[a,b]

| Position | 2 | 3 | 4 | 6 | 7 | 8 |
|---|---|---|---|---|---|---|
| 1 | 178.30 | 175.94 | 173.19 | 172.15 | 175.78 | 173.13 |
| 2 | 44.82 | 46.82 | 46.33 | 46.41 | 47.53 | 46.89 |
| 3 | 76.59 | 80.53 | 80.59 | 81.58 | 78.62 | 79.73 |
| 4 | 43.28 | 38.77 | 38.71 | 40.58 | 38.45 | 38.71 |
| 5 | 80.26 | 81.60 | 81.82 | 86.07 | 81.53 | 82.15 |
| 6 | 85.63 | 86.06 | 86.15 | 85.09 | 86.82 | 86.53 |
| 7 | 42.69 | 43.40 | 43.54 | 50.06 | 44.42 | 45.45 |
| 8 | 101.47 | 101.34 | 102.58 | 81.93 | 205.98 | 204.09 |
| 9 | 151.78 | 149.60 | 147.87 | 109.28 | 172.60 | 171.22 |
| 10 | 30.47 | 31.67 | 32.48 | 38.29 | 43.09 | 42.21 |
| 11 | 70.89 | 77.49 | 80.50 | 80.46 | 74.41 | 77.55 |
| 12 | 75.41 | 76.61 | 206.09 | 77.16 | NA | 206.12 |
| 13 | 78.28 | 76.70 | -- | 78.28 | 76.82 | -- |
| 13-$CH_2$ | 21.07 | 22.50 | -- | 22.79 | 22.86 | -- |
| 13-$CH_3$ | 10.58 | 11.82 | -- | 11.60 | 11.63 | -- |
| 2-$CH_3$ | 13.50 | 15.14 | 14.78 | 14.70 | 14.33 | 14.57 |
| 4-$CH_3$ | 8.72 | 9.29 | 9.42 | 10.81 | 9.37 | 9.84 |
| 6-$CH_3$ | 26.23 | 26.69 | 26.92 | 32.11 | 23.72 | 24.95 |
| 8-$CH_3$ | 11.83 | 10.93 | 10.95 | 23.96 | 32.80 | 32.10 |
| 10-$CH_3$ | 14.81 | 11.20 | 10.60 | 15.11 | 11.51 | 11.19 |
| 12-$CH_3$ | 16.17 | 15.56 | 27.42 | 18.03 | 17.98 | 26.98 |
| 1' | 102.99 | 103.99 | 103.97 | 105.03 | 102.91 | 103.47 |
| 2' | 70.48 | 71.06 | 71.06 | 70.30 | 72.24 | 70.82 |
| 3' | 65.88 | 65.37 | 65.44 | 65.39 | 76.15 | 65.47 |
| 4' | 28.83 | 28.83 | 28.95 | 28.90 | 34.77 | 28.92 |
| 5' | 68.81 | 68.97 | 69.00 | 69.51 | 67.41 | 69.15 |
| 5'-$CH_3$ | 21.30 | 21.18 | 21.25 | 20.94 | 20.98 | 21.25 |
| $N(CH_3)_2$ | 40.33 | 40.25 | 40.30 | 40.36 | 59.06/52.09 | 40.33 |
| 1" | 94.77 | 97.53 | 97.47 | 98.33 | 96.56 | 96.82 |
| 2" | 34.73 | 35.25 | 35.33 | 35.41 | 34.86 | 35.15 |
| 3" | 73.05 | 72.42 | 72.47 | 72.51 | 72.53 | 72.65 |
| 4" | 78.21 | 78.13 | 78.18 | 78.06 | 77.91 | 77.91 |
| 5" | 65.60 | 65.37 | 65.44 | 65.45 | 65.50 | 65.71 |

Table III. C-13 NMR Chemical Shifts of Macrolide Derivatives[a,b] (Continued)

| Position | 2 | 3 | 4 | 6 | 7 | 8 |
|---|---|---|---|---|---|---|
| 5"-$CH_3$ | 18.25 | 18.33 | 18.42 | 17.34 | 18.28 | 18.16 |
| 3"-$CH_3$ | 21.56 | 21.44 | 21.51 | 21.40 | 21.57 | 21.57 |
| 3"-$OCH_3$ | 49.50 | 49.30 | 49.36 | 49.08 | 49.48 | 49.36 |

a Obtained in deuteriochloroform solution using a Bruker WM-270 NMR spectrometer; chemical shifts are reported in parts per million using internal chlorform (77.0 pp.).

b Number of the carbon atoms in all compounds corresponds to their respective initial positions in 2.

c NA means not assigned.

Table IV: TLC and HPLC Data for Macrolide Derivatives[a,b]

| Compound Number[c] | TLC $R_f$ | HPLC $t_r$ (min) |
|---|---|---|
| 2 | 0.60 | 10.78 |
| 3 | 0.50 | 7.00 |
| 4 | 0.62 | 7.72 |
| 5 | 0.29 | 5.95 |
| 6 | 0.37 | 3.93 |
| 7 | 0.21 | 2.86 |
| 8 | 0.57 | 3.86 |

[a] TLC was performed using E. Merck plates of silicga gel 60 with a fluorescent indicate (F-254), dichloromethane-methanol-concentrated ammonium hydroxide (90:10:2) as the developing solvent and anisaldehyde-sulfuric acid spray reagent for detection.

[b] Analytical HPLC was performed on a Waters microbondapak C18 column with acetonitrile-methanol-1% ammonium acetate (30:30:40) as the mobile phase and a refrac-tive index detector.

## Claims

1. A compound of the formula (A)

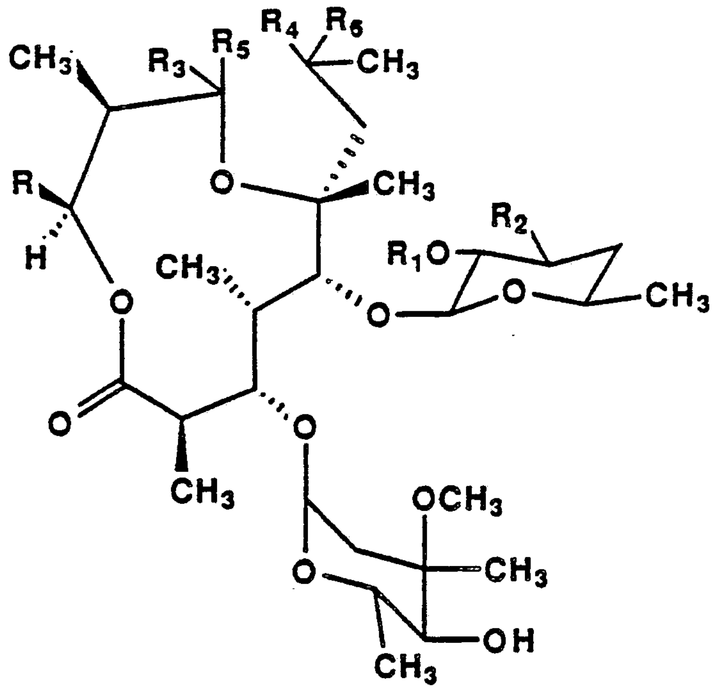

(A)

wherein

R is a)

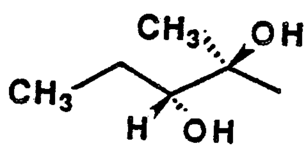

or b) acetyl;

$R^1$ is hydrogen or $C_1$-$C_5$-alkanoyl;
$R^2$ is $-N(CH_3)_2$ or $-N(CH_3)_2 \rightarrow O$; and
either

a) $R^5$ and $R^6$ taken together form a bond, and $R^3$ and $R^4$ are 1) either both hydroxyl or 2) taken together form a bond; or

b) each of $R^3$ and $R^5$ taken together and $R^4$ and $R^6$ taken together form a keto group;

provided that, when R is an (a) group and $R^2$ is $-N(CH_3)_2$, both $R^3$ and $R^4$ together and $R^5$ and $R^6$ together cannot form a bond; or a salt thereof.

2. A compound of claim 1 wherein $R^5$ and $R^6$ form a bond.

3. A compound of claim 1 wherein each of $R^3$ and $R^5$ taken together and $R^4$ and $R^6$ taken together form a keto group.

4. A compound of claim 1 or 2 wherein $R^3$ and $R^4$ together from a bond.

5. A compound of claim 1, 2, 3 or 4 wherein R is an (a) group.

6. A compound of claim 1, 2, 3 or 4 wherein R is acetyl.

7. A compound of claim 1, 2, 3, 4, 5 or 6 wherein $R^2$ is $-N(CH_3)_2$.

8. A pharmaceutical formulation which comprises as an active ingredient, a compound of formula 1, or a pharmaceutically acceptable salt thereof, as claimed in any one of claims 1 to 7, associated with one or more pharmaceutically acceptable carriers therefor.

9. A compound of formula 1, or a pharmaceutically acceptable salt thereof, as claimed in any one of claims 1 to 7, for use in inhibiting bacteria.

10. A process for preparing a macrolide of formula (A) as claimed in any one of Claims 1 to 7 which comprises:

reacting a macrolide of the formula (B)

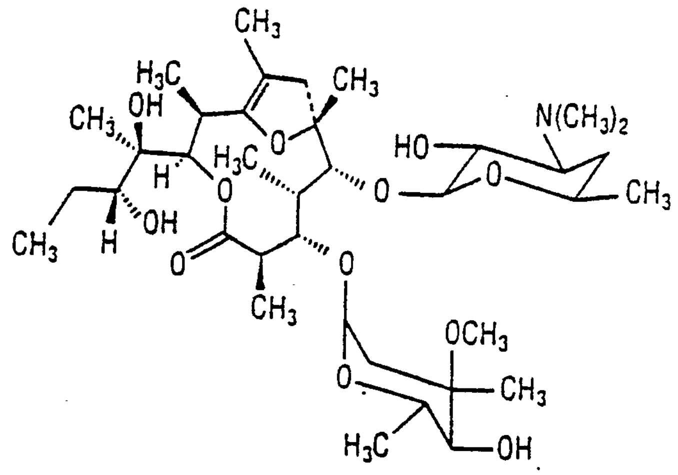

(B)

with

(a) an oxidizing agent such as lead tetraacetate or sodium periodate to form a formula (A) macrolide wherein R is acetyl; or

(b) an oxidizing agent such as hydrogen peroxide or a peracid to form a formula (A) macrolide wherein $R_2$ is $-N(CH_3)_2 \rightarrow O$; or

(c) an oxidizing agent such as bromine or an N-halosuccinimide to form a formula (A) macrolide wherein $R^3$ and $R^4$ are both hydroxyl; or

(d) an organic peroxy acid such as a haloperbenzoic acid to form a formula (A) macrolide wherein each of $R^3$ and $R^5$ taken together, and $R^4$ and $R^6$ taken together form a keto group; or

(e) an acylating agent to form a formula (A) macrolide wherein $R^1$ is $C_1$-$C_5$-alkanoyl.

Claims for the following Contracting States: GR, ES

1. A process for preparing a macrolide of formula (A) as claimed in any one of Claims 1 to 7 which comprises:

reacting a macrolide of the formula (B)

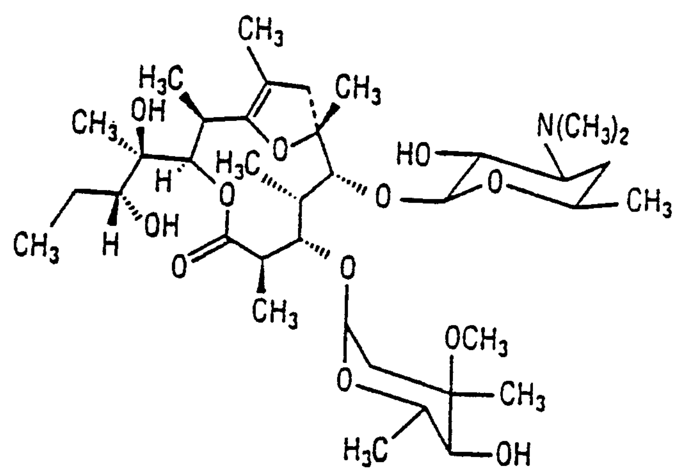

(B)

with

(a) an oxidizing agent such as lead tetraacetate or sodium periodate to form a formula (A) macrolide wherein R is acetyl; or

(b) an oxidizing agent such as hydrogen peroxide or a peracid to form a formula (A) macrolide wherein $R_2$ is $-N(CH_3)_2 \rightarrow O$; or

(c) an oxidizing agent such as bromine or an N-halosuccinimide to form a formula (A) macrolide wherein $R^3$ and $R^4$ are both hydroxyl; or

(d) an organic peroxy acid such as a haloperbenzoic acid to form a formula (A) macrolide wherein each of $R^3$ and $R^5$ taken together, and $R^4$ and $R^6$ taken together form a keto group; or

(e) an acylating agent to form a formula (A) macrolide wherein $R^1$ is $C_1$-$C_5$-alkanoyl.

2. A process for preparing a pharmaceutical formulation which comprises admixing a compound of formula (A), or a pharmaceutically acceptable salt thereof, as defined in Claim 1, with one or more pharmaceutically acceptable carriers therefor.

# FIG. 1

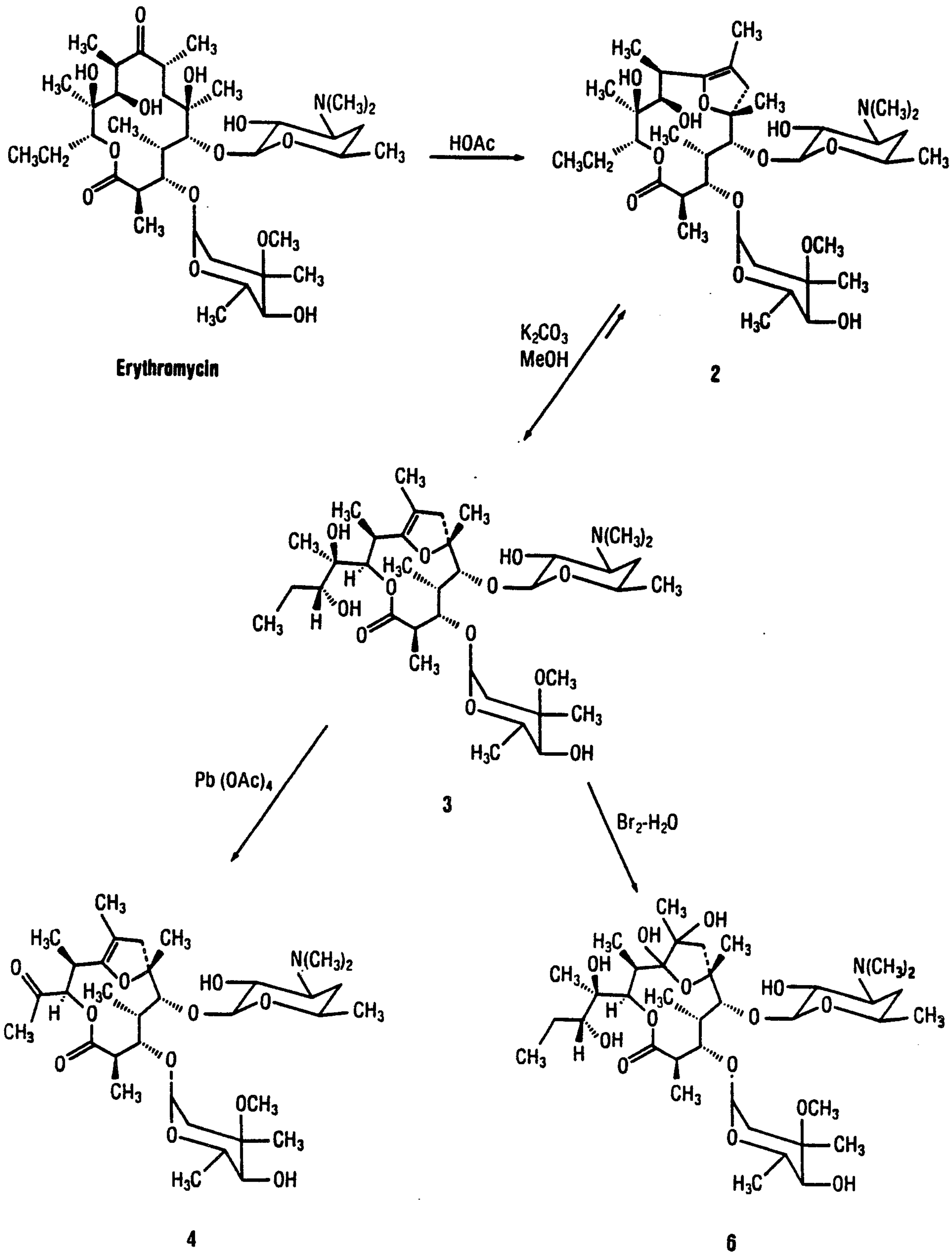


Erythromycin

2

3

4

6

# FIG. 2

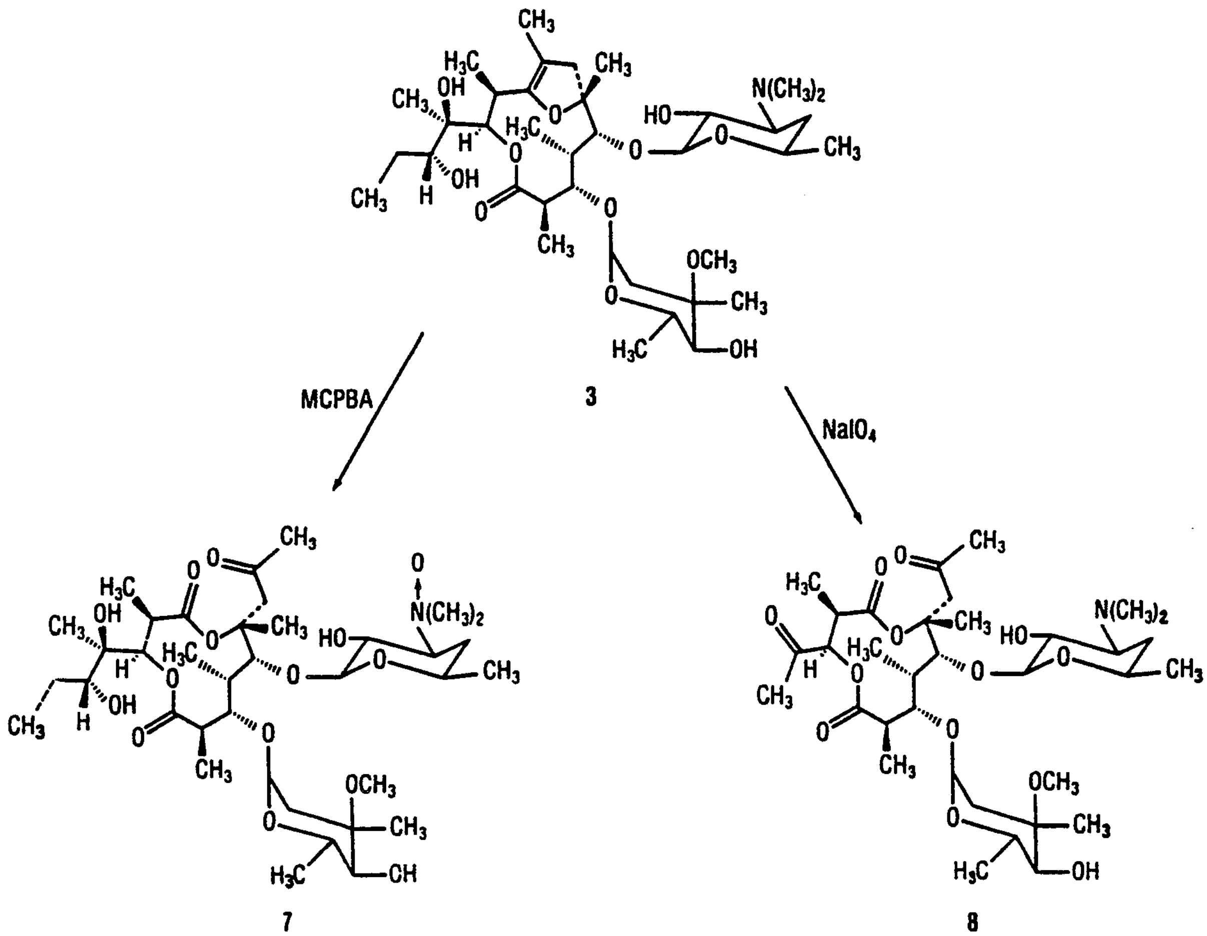


3

7

8